# EUROPÄISCHE PATENTSCHRIFT

(11) **EP 4 321 184 B1**
(45) Veröffentlichungstag und Bekanntmachung des Hinweises auf die Patenterteilung: **24.12.2025**
(21) Anmeldenummer: 23181113.4
(22) Anmeldetag: 23.06.2023
(51) Int. Cl.: A61M 1/00, A61M 3/02, B05B 1/08, B05B 9/04

(54) **LAVAGE-ANORDNUNG**
LAVAGE DEVICE
ENSEMBLE DE LAVAGE

(30) Priorität: 09.08.2022 DE 102022120056
(43) Veröffentlichungstag der Anmeldung: 14.02.2024
(73) Patentinhaber: H & B Electronic GmbH & Co. KG, 75392 Deckenpfronn (DE)
(72) Erfinder: Morlok, Tobias, 71159 Mötzingen (DE); Bayerbach, Jan, 75365 Calw (DE); Weinmann, Thomas, 75389 Neuweiler (DE)
(74) Vertreter: Wacker, Jost Oliver

(56) Entgegenhaltungen:
- EP-B1- 3 706 823
- US-A1- 2011 165 005
- US-A1- 2017 119 939

## Beschreibung

Die Erfindung betrifft eine Lavage-Anordnung zum Ausspülen und Reinigen von Körperstellen beziehungsweise Wunden mittels Sprüh- beziehungsweise Spülstößen während einer Operation, wie beispielsweise beim Einsetzen von künstlichen Gelenken oder Prothesen oder beim Wunddebridement. Die Lavage-Anordnung weist hierzu ein Applikatorgehäuse auf, das einen Handgriff und eine Applikationsöffnung zur Abgabe einer Spülflüssigkeit bildet, die insbesondere durch Wasser beziehungsweise eine wässrige Lösung mit darin enthaltenen Medikamenten und/oder Reinigungssubstanzen gebildet ist, wie beispielsweise eine NaCl-Lösung. Zudem umschließt das Applikatorgehäuse eine Antriebsaufnahme, die über eine Gehäuseöffnung zugänglich ist. Ferner weist die Lavage-Anordnung eine Flüssigkeitsleitung auf, mittels der die Applikationsöffnung mit einem Vorrat einer Spülflüssigkeit verbunden ist beziehungsweise verbunden werden kann. Zudem weist die Lavage-Anordnung eine in die Antriebsaufnahme einsetzbare Antriebseinheit einer Pumpe auf, die zur Förderung der Spülflüssigkeit vom Vorrat zur Applikationsöffnung dient. Hierzu weist die Antriebseinheit einen Motor, eine Stromversorgungseinrichtung und eine vom Motor antreibbare Pumpenantriebswelle auf.

Eine solche Lavage-Anordnung ermöglicht dabei ein schonendes und gründliches Spülen der betroffenen Operationsbereiche, insbesondere mittels wiederkehrender Spülstöße. Hierdurch können Knochen-, Gewebe- oder Materialrückstände und sonstige Verunreinigungen, die bei einer Operation anfallen, entfernt werden, wodurch wiederum Infektionen vermieden und Heilungsprozesse beschleunigt werden können.

Aus EP3706823 B1 ist eine Lavage-Anordnung bekannt, die eine Antriebseinheit mit einem Motor und einem Akkumulator aufweist. Die Antriebseinheit ist dabei auswechselbar in ein Gehäuse einsetzbar und weist eine Welle mit einem Antriebszahnrad auf. Im eingesetzten Zustand kämmt das Antriebszahnrad mit einem Pumpengetriebe einer Kolbenpumpe, die am Gehäuse gehalten ist.

Durch die auswechselbare Aufnahme der Antriebseinheit im Gehäuse kann letzteres als Einwegprodukt verwendet und nach einer erfolgten Anwendung aus hygienischen Gründen entsorgt werden, während die Antriebseinheit zusammen mit einem anderen, ungebrauchten Gehäuse wiederverwendet werden kann.

Nachteilig an der bekannten Lavage-Anordnung mit wiederverwendbarer Antriebseinheit ist, dass die Kosten und der Materialaufwand für das als Einwegteil konzipierte Gehäuse mit den daran gehaltenen Teilen, die für die Pumpfunktion benötigt werden, relativ hoch sind.

US 2011/165005 A1 beschreibt eine Pumpenvorrichtung mit einem aufklappbaren Gehäuse, in dem der Rotor einer Schlauchpumpe aufgenommen ist. Dabei kann zur Montage der Pumpenvorrichtung eine Schlauchanordnung, die einen rückverformbaren Abschnitt der Schlauchpumpe umfasst, derart in das Gehäuse eingesetzt werden, dass der rückverformbare Abschnitt beim Zuklappen des Gehäuses in eine betriebsbereite Stellung an Verdrängungsrollen des Rotors angelegt wird. Ein Motor zum Antrieb des Rotors ist dabei an einer vom klappbaren Gehäuse abgewandten Seite eines Trägers gehalten.

US 2017/0119939 A1 beschreibt eine Lavageanordnung zum Sprühen und Absaugen von Flüssigkeit mit einem Handstück, in dem eine Pumpe mit einem Impeller vorgesehen ist. In einer nur allgemein genannten Ausführungsvariante soll dabei eine Peristaltikpumpenanordnung verwendbar sein, bei der ein Sprühbetrieb oder ein Saugbetrieb in Abhängigkeit der Drehrichtung des Impellers auswählbar ist.

Die Aufgabe der Erfindung ist es, bei einer gattungsgemäßen Lavage-Anordnung die genannten Nachteile zu vermeiden und die Kosten und den Ressourcenverbrauch weiter zu senken.

Diese Aufgabe wird durch eine Lavage-Anordnung mit den Merkmalen des Anspruchs 1 gelöst. Dabei weist die Lavage-Anordnung einen mittels der Pumpenantriebswelle antreibbaren Rotor auf, an dem wenigstens ein Verdrängerelement gehalten ist, wobei der Rotor zusammen mit einem rückverformbaren Abschnitt der Flüssigkeitsleitung die Pumpe bildet und zwar in Form einer Schlauchpumpe. Durch diese Verwendung einer Schlauchpumpe zum Fördern der Spülflüssigkeit können die Herstellungskosten sowohl der Pumpe als auch der Lavage-Anordnung insgesamt deutlich reduziert werden. Zudem können die auf Seiten des Gehäuses für die Pumpfunktion benötigten Teile auf ein Minimum reduziert werden. Somit lassen sich insgesamt sowohl die Kosten als auch die bei wiederkehrender Benutzung der Lavage-Anordnung benötigten Ressourcen deutlich reduzieren.

Der Rotor ist dabei zusammen mit dem wenigstens einen Verdrängerelement vorzugsweise an der Antriebseinheit gehalten und mit dieser als Einheit austauschbar in die Antriebsaufnahme einsetzbar. Auf diese Weise können die für die Pumpenfunktion benötigten Teile zu einem sehr großen Teil wiederverwendet werden, während die für die Pumpenfunktion benötigten Teile, die nach einer Anwendung als Einwegelemente entsorgt werden, auf ein Minimum reduziert sind.

Gemäß der beanspruchten Erfindung ist der rückverformbare Abschnitt der Flüssigkeitsleitung am Applikatorgehäuse gehalten und der Rotor kann beim Einsetzen der Antriebseinheit in die Antriebsaufnahme an den rückverformbaren Abschnitt angelegt werden. Hierdurch kann zumindest der rückverformbare Abschnitt der Flüssigkeitsleitung als Einwegelement der Pumpe verwendet werden, das beim Entsorgen des Applikationsgehäuses mitentsorgt wird. Hierdurch kann sichergestellt werden, dass bei Wiederverwendung der Antriebseinheit mit einem noch unbenutzten Applikatorgehäuse auch ein unbenutzter rückverformbarer Abschnitt der Flüssigkeitsleitung vorliegt. Auf diese Weise kann bei der Wiederverwendung der Antriebseinheit mit einem jeweils neuen Applikatorgehäuse mit rückverformbaren Abschnitt einerseits die Keimfreiheit gewährleistet werden, als auch das Auftreten von Undichtigkeiten an der Schlauchpumpe in Folge von Verschleiß vermieden werden.

In einer besonders vorteilhaften Ausführungsform sind am Rotor wenigstens drei Verdrängerelemente gehalten. Hierdurch kann der rückverformbare Abschnitt der Flüssigkeitsleitung beim Antrieb des Rotors in relativ kurzen Abständen wiederkehrend zusammengepresst werden. Auf diese Weise werden in kurzen zeitlichen Abständen wiederkehrende Spülstöße aus der Applikationsöffnung heraus erzeugt.

Zudem ist es günstig, wenn die Verdrängerelemente wenigstens eine Rolle aufweisen. Vorzugsweise sind dabei alle Verdrängerelemente rollenförmig ausgebildet. Hierdurch wird ein gleichmäßiges und materialschonendes Auspressen des rückverformbaren Abschnittes der Flüssigkeitsleitung ermöglicht. Alternativ zur Rolle kann wenigstens ein Verdrängerelement als am Rotor unbeweglich gehaltenes Gleitelement gebildet sein, um beipielsweise den Wartungsaufwand zu verringern.

Dabei ist es günstig, wenn der rückverformbare Abschnitt der Flüssigkeitsleitung durch einen Schlauch mit abgerundetem Querschnitt, wie insbesondere einem kreisförmigen oder elliptischen Querschnitt gebildet ist, der an einen entprechend abgerundeten konkaven Anlagebereich des Applikatorgehäuses anlegbar ist. Hierdurch wird während des Zusammenpressens des rückverformbaren Abschnittes eine gleichmäßige flächige Anlage der Flüssigkeitsleitung am Applikatorgehäuse gewährleistet. Auf diese Weise können Beschädigungen an der Flüssigkeitsleitung während des Pumpvorganges vermieden werden.

Vorteilhafterweise weist das wenigstens eine Verdrängerelement des Rotors eine an den rückverformbaren Abschnitt anlegbare Kontaktfläche mit einem quer zu einer Förderrichtung ausgebildeten, abgerundeten Querprofil auf. Hierdurch ist ein im Wesentlichen vollständiges Zusammenpressen des rückverformbaren Abschnittes möglich, wodurch eine hohe Pumpleistung und starke Spülstöße generiert werden können.

Dabei ist es günstig, wenn die Gehäuseöffnung durch ein am übrigen Applikatorgehäuse beweglich gehaltenes Schließelement verschließbar ist. Auf diese Weise kann das Schließelement unverlierbar am übrigen Applikatorgehäuse gehalten werden, um letzteres sowohl vor und während der Verwendung der Lavage-Anordnung als auch bei der Entsorgung als einstückiges Einwegelement handhaben zu können.

Hierbei ist das Schließelement vorteilhafterweise durch einen Klappdeckel gebildet, wodurch die Applikationsöffnung des Applikatorgehäuses besonders einfach und schnell geöffnet beziehungsweise geschlossen werden kann, um die Antriebseinheit entnehmen beziehungsweise einbauen zu können.

Zudem ist es günstig, wenn das Applikatorgehäuse zusammen mit dem Schließelement aus einem einheitlichen Kunststoffmaterial hergestellt ist, um ein einfaches Recycling des Einwegteils zu ermöglichen.

Vorteilhafterweise kann an der Applikationsöffnung zudem ein Spülaufsatz, wie beispielsweise ein Trichter; ein Rohr bzw. eine Hohlnadel austauschbar angebracht werden, um einen für den jeweiligen Anwendungszweck geeigneten Sprühbeziehungsweise Spülstrahl generieren zu können. Zudem kann an dem Spülaufsatz zusätzlich eine Einsaugöffnung vorgesehen sein, über die die Spülflüssigkeit nach dem Ausspülen zusammen mit freigespülten Partikeln abtransportiert werden kann.

Der Vorrat der Spülflüssigkeit ist bevorzugterweise durch einen separaten Behälter gebildet, der über einen Versorgungsabschnitt der Flüssigkeitsleitung mit dem Applikatorgehäuse verbindbar ist. Hierdurch muss der Vorrat bei der Verwendung der Lavage-Anordnung nicht am Applikatorgehäuse mitgeführt werden und kann im Wesentlichen beliebig dimensioniert werden.

Vorzugsweise sind der Motor und die Stromversorgungseinrichtung in einem Antriebsgehäuse der Antriebseinheit geschützt aufgenommen. Das Antriebsgehäuse ist hierzu insbesondere wasserdicht ausgebildet, um eine Beschädigung des Motors und der Stromversorgungseinrichtung im Falle einer Reinigung oder Desinfektion der Antriebseinheit oder bei Austritt von Spülflüssigkeit zu vermeiden. Dadurch kann eine vielfache Wiederverwendung der Antriebseinheit ermöglicht werden.

Ferner ist die Stromversorgungseinrichtung vorteilhafterweise durch einen wiederaufladbaren Akkumulator gebildet, um die Stromversorgung der Antriebseinheit in einfacher Weise wiederkehrend zur Verfügung stellen zu können. Alternativ hierzu kann die Stromversorgungseinrichtung auch durch einen Anschluss eines Stromkabel gebildet sein, um eine im Wesentlichen unbegrenzte Stromversorgung zu ermöglichen.

Es wird darauf hingewiesen, dass alle oben beschriebenen Merkmale des erfindungsgemäßen Gegenstandes untereinander austauschbar beziehungsweise kombinierbar sind, sofern ein Austausch oder eine Kombination derselben aus technischen Gründen nicht ausgeschlossen ist.

In den Figuren ist eine beispielhafte Ausführungsform der Erfindung dargestellt. Es zeigen:
- Figur 1: eine Ansicht einer erfindungsgemäßen Lavage-Anordnung,
- Figur 2: eine explodierte Ansicht eines Applikators der Lavage-Anordnung nach Figur 1,
- Figur 3: einen Schnitt durch eine Schlauchpumpe des Applikators nach Figur 2 und
- Figur 4: eine teilweise geschnittene Draufsicht auf ein Verdrängerelement der Schlauchpumpe in Richtung IV aus Figur 3.

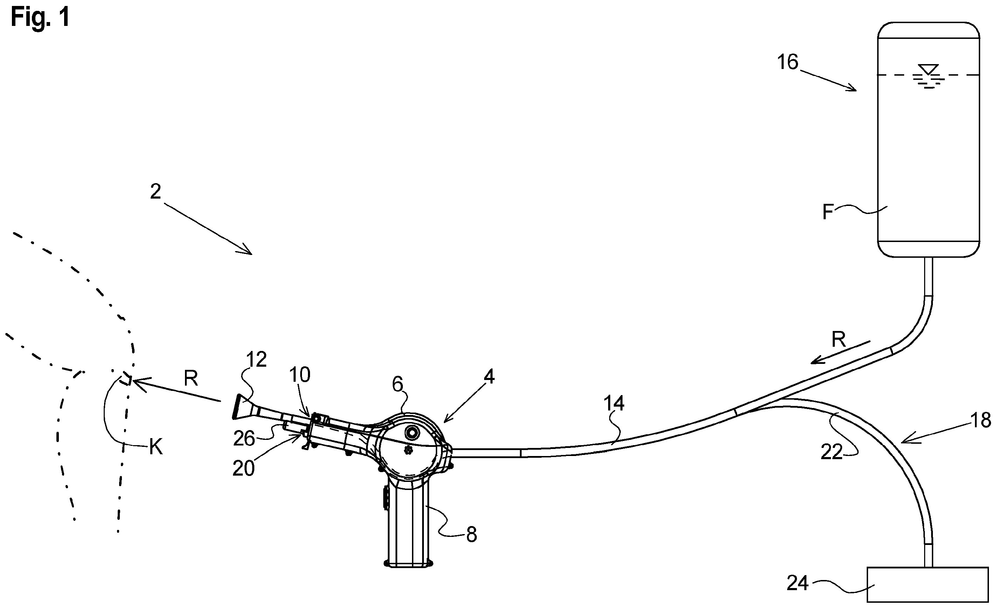

Fig. 1 zeigt eine Lavage-Anordnung 2 zum Ausspülen und Reinigen einer behandelten Körperstelle K in Form einer Wunde oder eines Knochens während einer Operation, wie beispielsweise beim Einsetzen eines künstlichen Gelenks oder einer Prothese. Hierzu weist die Lavage-Anordnung 2 einen Applikator 4 mit einem Applikatorgehäuse 6 auf. Dieses bildet einen Handgriff 8 und weist eine Applikationsöffnung 10 auf, an der ein Spülaufsatz 12 austauschbar angebracht werden kann. Je nach Anwendungsart kann dabei beispielsweise ein rohr- beziehungsweise hohlnadelförmiger oder, wie dargestellt, ein trichterförmiger Spülaufsatz 12 verwendet werden.

Über eine teilweise innerhalb des Applikatorgehäuses 6 verlegte Flüssigkeitsleitung 14 ist die Applikationsöffnung 10 mit einem separaten Vorratsbehälter 16 verbunden, in dem eine für den Spülvorgang verwendbare Spülflüssigkeit F bevorratet ist.

Ferner weist die Lavage-Anordnung 2 vorzugsweise eine Absauganordnung 18 mit einem Sauganschluss 20 auf, der ebenfalls an der Applikationsöffnung 10 vorgesehen sein kann und mit einem Absaugschlauch 22 verbunden ist. Der Absaugschlauch 22 kann dabei mit einem Unterdruck beaufschlagt werden, wie beispielsweise durch dessen Verbindung mit einer stationären Unterdruckeinrichtung 24. Die stationäre Unterdruckeinrichtung 24 kann dabei beispielsweise durch einen Chirurgiesauger eines Krankenhauses oder einer Arztpraxis gebildet sein.

Der dargestellte trichterförmige Spülaufsatz 12 kann dabei neben der Verbindung mit der Flüssigkeitsleitung 14 zudem einen Saugaufsatz 26 aufweisen, über den er mit dem Sauganschluss 20 verbunden werden kann. Der trichterförmige Spülaufsatz 12 kann somit während eines Spülvorganges auf die betreffende Körperstelle K aufgesetzt werden, um die abgegebene Spülflüssigkeit 16 innerhalb des Trichters wieder einsaugen und zusammen mit den beim Spülen abgelösten Partikeln über den Absaugschlauch 22 wegleiten zu können.

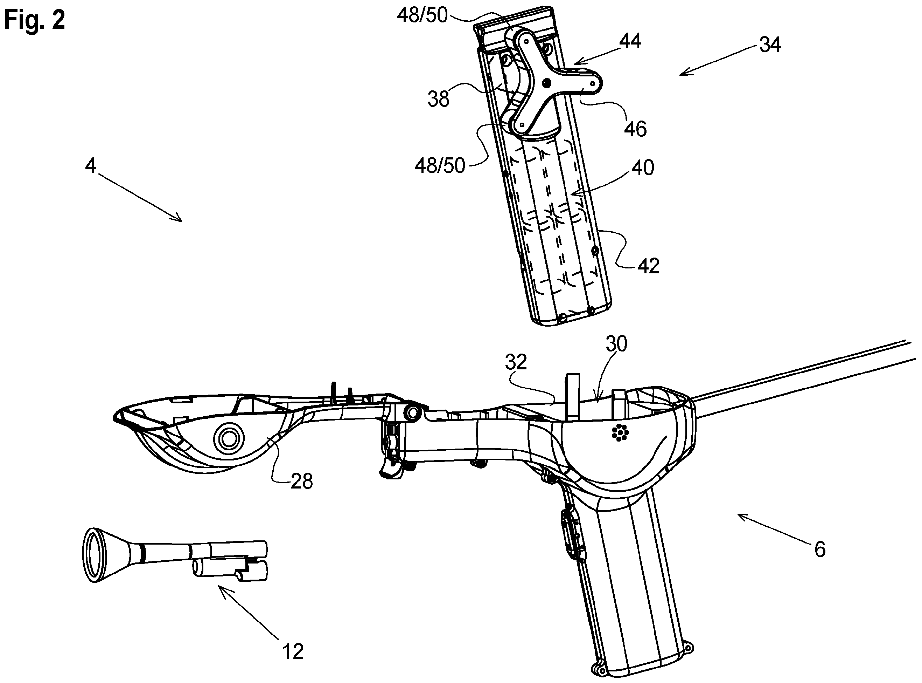

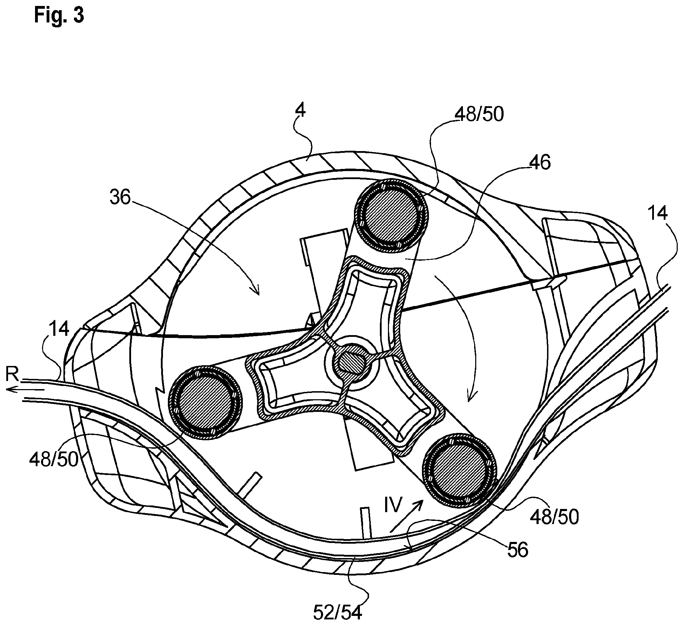

Wie insbesondere aus Figur 2 zu entnehmen ist, weist das Applikatorgehäuse 6 ein beweglich gehaltenes Schließelement 28 in Form eines Klappdeckels auf, der gegenüber dem übrigen Applikatorgehäuse 6 in eine dargestellte Offenstellung geklappt werden kann. In dieser Offenstellung ist eine Antriebsaufnahme 30 über eine Gehäuseöffnung 32 zugänglich. Die Antriebsaufnahme 30 dient dabei zur Aufnahme und Festlegung einer ausbaubaren Antriebseinheit 34 für eine Pumpe 36 (gemäß Figur 3). Hierzu weist die Antriebseinheit 34 einen Motor 38 und eine Stromversorgungseinrichtung 40 in Form eines, vorzugsweise wiederaufladbaren Akkumulators auf. Alternativ zur Verwendung eines Akkumulators kann die Stromversorgungseinrichtung 40 auch durch einen Anschluss eines Stromkabels gebildet sein. In jedem Fall sind der Motor 38 und die Stromversorgungseinrichtung 40 dabei geschützt in einem vorzugsweise wasserdichten Antriebsgehäuse 42 aufgenommen, das ein vielfaches Reinigen und Desinfizieren der Antriebseinheit 34 ohne Beschädigung des Motors 38 und der Stromversorgungseinrichtung 40 erlaubt.

An der Außenseite des Antriebsgehäuses 42 ist eine vom Motor 38 antreibbare Pumpenantriebswelle 44 vorgesehen. An dieser ist ein Rotor 46 gehalten, an dem wenigstens ein Verdrängerelement 48, vorzugsweise wenigstens drei umlaufend angeordnete Verdrängerelemente 48 gelagert sind. Die Verdrängerelemente 48 sind dabei beispielhaft durch verdrehbar am Rotor 46 gelagerte Rollen 50 gebildet. Alternativ hierzu können die Verdrängerelemente 48 auch durch fest mit dem Rotor 46 verbundene Gleitelemente gebildet sein (nicht dargestellt).

In jedem Fall dienen die Verdrängerelemente 48 zusammen mit einem rückverformbaren Abschnitt 52 der Flüssigkeitsleitung 14, gemäß Figur 3, zur Ausbildung der Pumpe 36 als Schlauchpumpe. Der rückverformbare Abschnitt 52 ist dabei durch einen Schlauch 54 mit einem kreisrunden oder elliptischen Querschnitt gebildet, der an einem konkaven Anlegebereich 56 an der Innenseite des Applikatorgehäuses 6 geführt beziehungsweise gehalten ist.

Die Schlauchpumpe 36 ist durch den vom Motor 38 angetriebenen Rotor 46 aktivierbar, um die Spülflüssigkeit F entlang der Förderrichtung R vom Vorratsbehälter 16 über die Flüssigkeitsleitung 14 zum Applikator 4 zu fördern und an der Applikationsöffnung 10 beziehungsweise an dem daran aufgesetzten Spülaufsatz 12 in wiederkehrenden Spülstößen abzugeben.

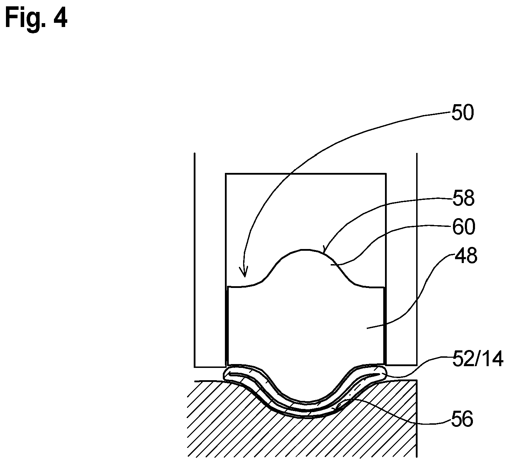

Hierzu wird der rückverformbare Abschnitt 52 nach Figur 3 bei der Drehbewegung des Rotors 46 aufeinanderfolgend und wiederkehrend von dem wenigstens einen Verdrängerelementen 48 beaufschlagt. Diese pressen dabei den rückverformbaren Abschnitt 52 jeweils zusammen und bewegen sich gleichzeitig in Förderrichtung R, wodurch auch die in der Flüssigkeitsleitung 14 enthaltene Spülflüssigkeit F in Förderrichtung R transportiert wird. Je nachdem ob die Verdrängerelemente 48 dabei als Rollen 50 oder Gleitelemente ausgebildet sind, rollen oder gleiten diese auf dem Schlauch. Um hierbei ein möglichst vollständiges Zusammenpressen des rückverformbaren Abschnittes 52 zu ermöglichen, weisen die Verdrängerelemente 48 an einer Kontaktfläche 58 ein zum konkaven Anlegebereich 56 im Wesentlichen komplementär ausgebildetes Querprofil 58 auf, wie in Figur 4 beispielhaft für eine der Rollen 50 dargestellt ist.

Nach einer Verwendung der Lavage-Anordnung 2 gemäß Figur 1 kann die Antriebseinheit 34, die während des Betriebs spritzgeschützt im Applikatorgehäuse 6 aufgenommen war, nach Öffnen des Schließelementes 28 aus der Antriebsaufnahme 30 entnommen und zur Wiederverwendung in ein unbenutztes Applikatorgehäuse 6 eingesetzt werden. Das bereits verwendete Applikatorgehäuse 6, das an seiner Außenseite in der Regel Verunreinigungen in Folge der während des Betriebs erzeugten Spülstöße aufweist, kann dagegen als Einwegelement aus hygienischen Gründen entsorgt werden. Zur leichteren Entsorgung beziehungsweise für ein effizienteres Recycling des Applikatorgehäuses 6, ist dieses einschließlich des Schließelementes 28 dabei vorzugsweise aus einem einheitlichen Kunststoff hergestellt. Ein bei der erfolgten Anwendung verwendeter Spülaufsatz 12 und zumindest der rückverformbare Abschnitt 52 der Flüssigkeitsleitung 14, können zudem als weitere Einwegelemente entsorgt werden.

Vor der Wiederverwendung der Antriebseinheit 34 mit dem noch unbenutzten neuen Applikatorgehäuse 6 kann zudem die durch einen Akkumulator gebildete Stromversorgungseinrichtung 40 aufgeladen oder ausgewechselt werden. Hierdurch kann bei der Wiederverwendung der Antriebseinheit 34 die volle Leistung des Motors 38 und der Pumpe 36 und der Lavage-Anordnung insgesamt gewährleistet werden.

Es wird darauf hingewiesen, dass alle oben beschriebenen Elemente und Merkmale der verschiedenen Ausführungsformen untereinander austauschbar beziehungsweise kombinierbar sind, sofern ein Austausch oder eine Kombination derselben aus technischen Gründen nicht ausgeschlossen ist. Die Erfindung wird durch die Merkmale des unabhängigen Anspruchs 1 definiert.

## Patentansprüche

1. Lavage-Anordnung (2)
mit einem Applikatorgehäuse (6), das einen Handgriff (8) und eine Applikationsöffnung (10) zur Abgabe einer Spülflüssigkeit (F) aufweist und eine Antriebsaufnahme (30) umschließt, die über eine Gehäuseöffnung (32) zugänglich ist,
mit einer Flüssigkeitsleitung (14), mittels der die Applikationsöffnung (10) mit einem Vorrat (16) der Spülflüssigkeit (F) verbindbar ist,
und mit einer in die Antriebsaufnahme (30) einsetzbaren Antriebseinheit (34) einer Pumpe (36) zur Förderung der Spülflüssigkeit (F) vom Vorrat (16) zur Applikationsöffnung (10), wobei die Antriebseinheit (34) hierzu einen Motor (38), eine Stromversorgungseinrichtung (40) und eine vom Motor (38) antreibbare Pumpenantriebswelle (44) aufweist,
wobei ein mittels der Pumpenantriebswelle (44) antreibbarer Rotor (46) mit wenigstens einem Verdrängerelement (48) vorgesehen ist, der zusammen mit einem rückverformbaren Abschnitt (52) der Flüssigkeitsleitung (14) die Pumpe (36) in Form einer Schlauchpumpe (36) bildet, wobei der Rotor (46) mit dem wenigstens einen Verdrängerelement (48) an der Antriebseinheit (34) gehalten und mit dieser austauschbar in die Antriebsaufnahme (30) einsetzbar ist,
**dadurch gekennzeichnet, dass** die Flüssigkeitsleitung (14) am Applikatorgehäuse (6) gehalten ist, wobei der Rotor (46) beim Einsetzen der Antriebseinheit (34) in die Antriebsaufnahme (30) an den rückverformbaren Abschnitt (52) anlegbar ist.

2. Lavage-Anordnung nach Anspruch 1, **dadurch gekennzeichnet, dass** am Rotor (46) wenigstens drei Verdrängerelemente (48) gehalten sind.

3. Lavage-Anordnung nach Anspruch 1oder 2, **dadurch gekennzeichnet, dass** die Verdrängerelemente (48) wenigstens eine Rolle (50) aufweisen.

4. Lavage-Anordnung nach einem der Ansprüche 1 bis 3, **dadurch gekennzeichnet, dass** der rückverformbare Abschnitt (52) der Flüssigkeitsleitung (14) durch einen Schlauch mit abgerundetem Querschnitt gebildet ist, der an einen entprechend abgerundeten konkaven Anlagebereich (56) des Applikatorgehäuses (6) anlegbar ist.

5. Lavage-Anordnung nach Anspruch 4, **dadurch gekennzeichnet, dass** das wenigstens eine Verdrängerelement (48) eine an den rückverformbaren Abschnitt (52) anlegbare Kontaktfläche (58) mit einem quer zu einer Förderrichtung (R) ausgebildeten konkaven, abgerundeten Querprofil (60) aufweist.

6. Lavage-Anordnung nach einem der Ansprüche 1 bis 5, **dadurch gekennzeichnet, dass** die Gehäuseöffnung (32) durch ein am übrigen Applikatorgehäuse (6) beweglich gehaltenes Schließelement (28) verschließbar ist.

7. Lavage-Anordnung nach Anspruch 6, **dadurch gekennzeichnet, dass** das Schließelement (28) durch einen Klappdeckel gebildet ist.

8. Lavage-Anordnung nach Anspruch 6 oder 7, **dadurch gekennzeichnet, dass** das Applikatorgehäuse (6) mit dem Schließelement (28) aus einem einheitlichen Kunststoffmaterial hergestellt ist.

9. Lavage-Anordnung nach einem der Ansprüche 1 bis 8, **dadurch gekennzeichnet, dass** an der Applikationsöffnung (10) ein Spülaufsatz (12) austauschbar anbringbar ist.

10. Lavage-Anordnung nach einem der Ansprüche 1 bis 9, **dadurch gekennzeichnet, dass** der Vorrat (16) der Spülflüssigkeit (F) durch einen separaten Behälter gebildet ist, der über die Flüssigkeitsleitung (14) mit dem Applikatorgehäuse (6) verbindbar ist.

11. Lavage-Anordnung nach einem der Ansprüche 1 bis 10, **dadurch gekennzeichnet, dass** der Motor (38) und die Stromversorgungseinrichtung (40) in einem Antriebsgehäuse (42) der Antriebseinheit (34) geschützt aufgenommen sind.

12. Lavage-Anordnung nach einem der Ansprüche 1 bis 11, **dadurch gekennzeichnet, dass** die Stromversorgungseinrichtung (40) durch einen wiederaufladbaren Akkumulator gebildet ist.

## Claims

1. Lavage Arrangement (2)
with an applicator housing (6) having a handle (8) and an application opening (10) for dispensing a rinsing fluid (F) and enclosing a drive receptacle (30) which is accessible via a housing opening (32),
with a liquid line (14), by means of which the application opening (10) can be connected to a reservoir (16) of the rinsing fluid (F),
and with a drive unit (34) of a pump (36) for pumping the rinsing fluid (F) from the reservoir (16) to the application opening (10) which can be inserted into the drive receptacle (30), wherein the drive unit (34) for this purpose comprises a motor (38), a power supply device (40) and a pump drive shaft (44) which can be driven by the motor (38),
wherein a rotor (46) which can be driven by means of the pump drive shaft (44) with at least one displacement element (48) is provided, which together with a deformable section (52) of the liquid line (14) forms the pump (36) in the form of a peristaltic pump (36), wherein the rotor (46) with at least one displacement element (48) is held on the drive unit (34) and can be used interchangeably with this in the drive receptacle (30),
**characterized in that** the liquid line (14) is held to the applicator housing (6), wherein the rotor (46) can be attached to the reformable section (52) when the drive unit (34) is inserted into the drive receptacle (30).

2. Lavage arrangement according to claim 1, **characterized in that** at least three displacement elements (48) are held on the rotor (46).

3. Lavage arrangement according to claim 1 or 2, **characterized in that** the displacement elements (48) have at least one roller (50).

4. Lavage arrangement according to any one of claims 1 to 3, **characterized in that** the deformable section (52) of the liquid line (14) is formed by a tubing with a rounded cross-section, which is attachable to a correspondingly rounded concave contact area (56) of the applicator housing (6).

5. Lavage arrangement of claim 4, **characterized in that** the at least one displacement element (48) has a contact surface (58) which can be applied to the deformable section (52) with a concave, rounded transverse profile (60) formed transversely to a conveying direction (R).

6. Lavage arrangement according to any one of claims 1 to 5, **characterized in that** the housing opening (32) can be closed by a closing element (28) held movably on the remainder of the applicator housing (6).

7. Lavage arrangement according to claim 6, **characterized in that** the closing element (28) is formed by a hinged cover.

8. Lavage arrangement according to claim 6 or 7, **characterized in that** the applicator housing (6) with the closing element (28) is made of a single plastic material.

9. Lavage arrangement according to any one of claims 1 to 8, **characterized in that** a rinsing attachment (12) can be attached interchangeably to the application opening (10).

10. Lavage arrangement according to any one of claims 1 to 9, **characterized in that** the reservoir (16) of the rinsing fluid (F) is formed by a separate container which is connectable to the applicator housing (6) via the liquid line (14).

11. Lavage arrangement according to any one of claims 1 to 10, **characterized in that** the motor (38) and the power supply device (40) are contained in a drive housing (42) of the drive unit (34) in a protected manner.

12. Lavage arrangement according to any one of claims 1 to 11, **characterized in that** the power supply device (40) is formed by a rechargeable battery.

## Revendications

1. Dispositif de lavage (2)
avec un boîtier d'applicateur (6) ayant une poignée (8) et une ouverture d'application (10) pour la distribution d'un liquide de rinçage (F) et renfermant un support d'entraînement (30) accessible par une ouverture de boîtier (32),
avec une conduite de liquide (14), au moyen de laquelle l'ouverture d'application (10) peut être connectée à une alimentation (16) du liquide de rinçage (F),
et avec une unité d'entraînement (34) d'une pompe (36) pour pomper le fluide de rinçage (F) de l'alimentation (16) à l'ouverture d'application (10) qui peut être insérée dans le support d'entraînement (30), l'unité d'entraînement (34) à cet effet comprenant un moteur (38), un dispositif d'alimentation (40) et un arbre d'entraînement de pompe (44) qui peut être entraîné par le moteur (38),
dans lequel est prévu un rotor (46) qui peut être entraîné au moyen de l'arbre d'entraînement de pompe (44) avec au moins un élément de déplacement (48), qui, avec une partie déformable (52) de la conduite de liquide (14), forme la pompe (36) sous la forme d'une pompe péristaltique (36), dans lequel le rotor (46) avec au moins un élément de déplacement (48) est maintenu sur l'unité d'entraînement (34) et peut être utilisé de manière interchangeable avec celui-ci dans le support d'entraînement (30),
**caractérisé en ce que** la conduite de liquide (14) est maintenue au boîtier de l'applicateur (6), dans lequel le rotor (46) peut être fixé à la partie déformable (52) lorsque l'unité d'entraînement (34) est insérée dans le support d'entraînement (30).

2. Dispositif de lavage selon la revendication 1, **caractérisé en ce qu'**au moins trois éléments de déplacement (48) sont maintenus sur le rotor (46).

3. Dispositif de lavage selon la revendication 1 ou 2, **caractérisé en ce que** les éléments de déplacement (48) comportent au moins un rouleau (50).

4. Dispositif de lavage selon l'une quelconque des revendications 1 à 3, **caractérisé en ce que** la partie déformable (52) de la conduite de liquide (14) est formée par un tube de section arrondie, qui peut être fixé à une zone d'application concave (56) arrondie correspondante du boîtier de l'applicateur (6).

5. Dispositif de lavage selon la revendication 4, **caractérisé en ce que** le ou les éléments de déplacement (48) ont une surface de contact (58) qui peut être appliquée sur la partie déformable (52) avec une section transversale concave et arrondie (60) formée transversalement à une direction de transport (R).

6. Dispositif de lavage selon l'une quelconque des revendications 1 à 5, **caractérisé en ce que** l'ouverture du boîtier (32) peut être fermée par un élément de fermeture (28) maintenu mobile sur le reste du boîtier de l'applicateur (6).

7. Dispositif de lavage selon la revendication 6, **caractérisé en ce que** l'élément de fermeture (28) est formé par un couvercle à charnière.

8. Dispositif de lavage selon la revendication 6 ou 7, **caractérisé en ce que** le boîtier de l'applicateur (6) avec l'élément de fermeture (28) est constitué d'une matière plastique uniforme.

9. Dispositif de lavage selon l'une quelconque des revendications 1 à 8, **caractérisé en ce qu'**un accessoire affleurant (12) peut être fixé de manière interchangeable à l'ouverture d'application (10).

10. Dispositif de lavage selon l'une quelconque des revendications 1 à 9, **caractérisé en ce que** l'alimentation (16) du fluide d'irrigation (F) est formée par un récipient séparé qui peut être raccordé au boîtier de l'applicateur (6) via la conduite de liquide (14).

11. Dispositif de lavage selon l'une quelconque des revendications 1 à 10, **caractérisé en ce que** le moteur (38) et le dispositif d'alimentation (40) sont contenus de manière protégée dans un boîtier d'entraînement (42) de l'unité d'entraînement (34).

12. Dispositif de lavage selon l'une quelconque des revendications 1 à 11, **caractérisé en ce que** le dispositif d'alimentation (40) est formé par un accumulateur rechargeable.
